# EUROPEAN PATENT SPECIFICATION

(11) **EP 1 860 142 B1**
(45) Date of publication and mention of the grant of the patent: **17.10.2012**
(21) Application number: 06715519.2
(22) Date of filing: 10.03.2006
(51) Int. Cl.: C08J 9/28, C12N 11/08, C12N 5/00

(54) **PROCESS FOR PRODUCING POROUS OBJECT AND POROUS OBJECT OBTAINED BY THE SAME**
HERSTELLUNGSVERFAHREN FÜR EIN PORÖSES OBJEKT UND MIT DIESEM VERFAHREN HERGESTELLTES PORÖSES OBJEKT
PROCEDE DE PRODUCTION D UN OBJET POREUX ET OBJET POREUX OBTENU PAR L INTERMEDIAIRE DE CE PROCEDE

(30) Priority: 18.03.2005 JP 2005080059
(43) Date of publication of application: 28.11.2007
(73) Proprietor: JMS Co., Ltd., Hiroshima-shi, Hiroshima 730-8652 (JP)
(72) Inventor: SAJIKI, Toshinobu, Hiroshima-shi, Hiroshima 730-0005 (JP); IDE, Junichi, Hiroshima-shi, Hiroshima 780-0843 (JP); HANAKI, Naoyuki, Osaka 569-1044 (JP); MATSUURA, Yoji, Hiroshima-shi, Hiroshima 733-0034 (JP)
(74) Representative: Isarpatent
(86) International application number: PCT/JP2006/304699
(87) International publication number: WO 2006/100935

(56) References cited:
- WO-A1-00/62829
- JP-A- 02 265 935
- JP-A- 10 234 844
- JP-A- 2001 049 018
- JP-A- 2004 216 119
- US-A- 6 051 618
- US-A1- 2003 181 978
- US-B1- 6 281 257
- DATABASE WPI Week 200474 Thomson Scientific, London, GB; AN 2004-754609 XP002531078 & KR 2004 058 572 A (KOREA ADV INST SCI & TECHNOLOGY) 5 July 2004 (2004-07-05)
- DE GROOT J H; PENNINGS A J; COENEN J M F H: "Triple-layer artificial skin: Porous 50/50 copoly (L-lactide/[epsilon]-caprolactone) template for neodermis regeneration" JOURNAL OF MATERIALS SCIENCE LETTERS, vol. 16, 1997, pages 152-154, XP002531076
- DATABASE WPI Week 200051 Thomson Scientific, London, GB; AN 2000-551712 XP002531079 & JP 2000 197693 A (GUNZE KK) 18 July 2000 (2000-07-18)
- DATABASE WPI Week 200518 Thomson Scientific, London, GB; AN 2005-168508 XP002531080 & JP 2005 046538 A (NIPPON MEDICAL SUPPLY KK) 24 February 2005 (2005-02-24)
- MAQUET V; BLACHER S; PIRARD R; PIRARD J-P; VYAKARNAM M N; JEROME R: "Preparation of macroporous biodegradable poly(L-lactide-co-[epsilon] -caprolactone) foams and characterization by mercury intrusion porosimetry, image analysis, and impedance spectroscopy" JOURNAL OF BIOMEDICAL MATERIALS RESEARCH - PART A, vol. 66a, 2003, pages 199-213, XP009118001

## Description

### Technical Field

The present invention relates to processes for producing porous objects, particularly those that are useful as scaffold materials for cells in medical fields, especially tissue engineering and regenerative medical engineering.

### Background Art

In the fields of tissue engineering and regenerative medical engineering, scaffold materials generally are used for cell proliferation. Particularly recently, it is expected that porous objects formed of bioabsorbable materials are used as scaffold materials. When such a bioabsorbable porous object is used, cells are seeded and proliferated in pores thereof, and then it is transplanted into a biological body. This allows tissue regeneration to occur in the biological body, while the bioabsorbable material serving as a scaffold is decomposed and absorbed gradually in the biological body. This makes it possible to transplant the scaffold used for proliferating cells into the biological body together with proliferating cells.

A freeze-drying method is used widely as a method for producing such porous objects. For example, a method in which a bioabsorbable polymer is dissolved in a dioxane solvent, which then is freeze-dried to be porosified, is disclosed as a general method (see, for instance, Patent Document 1). However, the porous objects obtained by this method have pore sizes of 100 µm or smaller. Accordingly, it is difficult to obtain porous objects with pore sizes of 100 µm or larger that are suitable for cells to penetrate into the porous objects, for example.

Patent Document 5 provides a biomedical, biocompatible, polymeric foam scaffold suitable for use in the repair and regeneration of tissue and to methods for making such biomedical scaffolds. A method for producing the porous object from a PLA/PCL copolymer by using dimethyl carbonate and t-butanol is described therein which requires three cycles of cooling. However, the porous objects obtained by this method comprise a network of branched dendritic channels and not uniform pores. Furthermore, methods of porosifying by adding particles of sodium chloride, sugar, etc. to a polymer solution, freeze-drying it, and then eluting and removing the particles through washing with water also are proposed, for example (see, for instance, Patent Document 2 and Patent Document 3). With these methods, since portions where the particles were present become pores, porous objects can be obtained that have pores whose sizes are approximately the same as those of the particles used. However, such methods have the following problems. That is, the production process is complicated since the elution of the particles is required, and it is difficult to secure uniformity in pore distribution in the porous objects to be obtained, due to sedimentation of the particles in the polymer solution. Furthermore, in order to improve the uniformity of pore size, it is necessary to use particles whose diameters are uniform, which results in an increase in cost. Since it is difficult to remove the particles completely, there is a possibility that the particles may remain in the porous objects. Thus, it is very difficult to obtain desired pore sizes, particularly larger pore sizes (several hundreds of micrometers), in producing porous objects.

In addition, a method of controlling pore sizes by adding water and an organic solvent that is compatible with water and thereby adjusting the rate between the both to be added when a collagen solution is freeze-dried has been disclosed as a method of controlling pore sizes (for instance, Patent Document 4). However, the pore sizes obtained by this method are about 50 to 80 µm and it is difficult to obtain pore sizes over a wide range by this method. Furthermore, a quick freezing method using liquid nitrogen is used generally as a freezing method, but this method tends to result in smaller pore sizes.
[Patent Document 1: JP10(1998)-234844A]
[Patent Document 2: JP2001-49018A]
[Patent Document 3: JP2002-541925A]
[Patent Document 4: JP02(1990)-265935A]
[Patent Document 5: US 2003/0181978A1]

### Disclosure of Invention

### Problem to be Solved by the Invention

Hence, an object of the present invention is to provide a process for producing porous objects that makes it possible to adjust pore sizes, particularly not only smaller pore sizes but also larger pore sizes.

### Means for Solving the Problem

The process for producing a porous object of the present invention includes: preparing a mixed solution; freeze-treating the mixed solution; and drying the mixed solution that has been freeze-treated, under reduced pressure. The mixed solution contains: a polymer containing a copolymer of lactide and caprolactone; a solvent in which the polymer has a relatively low solubility; and a solvent in which the polymer has a relatively high solubility and that is compatible with the solvent in which the polymer has a relatively low solubility. The process is characterized in that the pore size of the porous object is controlled by varying the content of the solvent in which the polymer has a relatively low solubility in the mixed solution in the process of preparing the mixed solution, and cooling the mixed solution at a rate of 300°C/hr or lower in the process of freeze-treating. Hereinafter, the solvent in which the polymer has a relatively low solubility is referred to as a "poor solvent", while the solvent in which the polymer has a relatively high solubility is referred to as a "good solvent". However, in the present invention, these terms are used merely for differentiating the two according to the relative solubility of the polymer.

### Effect of the Invention

The process for producing a porous object of the present invention makes it possible to adjust pore sizes easily over a wide range and to form relatively uniform pores by varying the content of the poor solvent in the mixed solution and cooling the mixed solution at a rate of 300°C/hr or lower to freeze the mixed solution.

As described above, it has been known to adjust pore sizes by varying the ratio between the poor solvent such as water and the good solvent such as an organic solvent (Patent Document 4). However, the present invention makes it possible to obtain a wide range of pore sizes, namely 30 to 1800 µm, for example, and to obtain uniformity of pores to be formed, by additionally setting the cooling rate to be employed in freeze-treating the mixed solution to a rate of 300°C/hr or lower. That is, the present inventors found out that even in the case of using mixed solutions containing the same content of the poor solvent, when the setting of the cooling rate (300°C/hr or lower) is varied, the size of pores to be formed can be changed further. As a result, the present inventors made it possible to broaden the range of pore sizes further by combining the adjustment of the content and the setting of the cooling rate. These facts that in the above-mentioned manner, the pore sizes can be set over a wide range and particularly a pore size of 100 µm or more also can be obtained were found out by the present inventors for the first time. The pore sizes of porous objects obtained by conventional methods in which no particles are used are generally 10 to 80 µm. From this, it also can be said that the present invention allows the pore sizes to be set over a very wide range. Furthermore, since no particles are mixed as described above, there are no problems such as uneven distribution of particles caused by sedimentation, remaining particles, etc. Accordingly, the present invention makes it possible to obtain various pore sizes by merely setting the content of the poor solvent and the cooling temperature. For instance, pore sizes suitable for the intended uses of the porous objects can be obtained. Thus, it can be said that the process for producing a porous object of the present invention is highly useful in tissue engineering, regenerative medicine, etc.

### Brief Description of Drawings

FIG. 1 is a graph showing the relationship between the water content in mixed solutions and the pore size of porous objects obtained in an example of the present invention.
FIG. 2 is a graph showing the relationship between the water content in mixed solutions and the pore size of porous objects obtained in another example of the present invention.
FIG. 3 is a graph showing the relationship between the water content in mixed solutions and the pore size of porous objects obtained in still another example of the present invention.
FIG. 4 is a photograph showing a cross section of a porous object according to the above-mentioned example.
FIG. 5 shows photographs indicating the results of cell staining after the porous objects each are implanted in a biological body in yet another example of the present invention.

### Best Mode for Carrying Out the Invention

As described above, the present invention provides a process for producing a porous object that includes: preparing a mixed solution; freeze-treating the mixed solution; and drying the mixed solution that has been freeze-treated, under reduced pressure. In the process of freeze-treating the mixed solution, the mixed solution is cooled at a constant rate of 300°C/hr or lower and thereby is frozen. The mixed solution contains: a polymer containing a copolymer of lactide and caprolactone; a poor solvent with respect to the aforementioned polymer; and a good solvent with respect to the aforementioned polymer that is compatible with the poor solvent. The poor solvent in which the polymer has a relatively low solubility, is at least one selected from the group consisting of water, ethanol, tertiary butyl alcohol (tBuOH); and the good solvent in which the polymer has a relatively high solubility, is at least one of 1,4-dioxane and dimethyl carbonate. The process is characterized in that the pore size of the porous object is controlled by varying the content of the solvent in which the polymer has a relatively low solubility in the mixed solution in the process of preparing the mixed solution, and cooling the mixed solution at a rate of 300°C/hr or lower in the process of freeze-treating, e.g. at a constant rate of 300°C/hr or lower.

The copolymer to be used in the present invention is a copolymer of lactide and caprolactone as described above. It can be either a random polymer or a block polymer. Furthermore, a mixture of at least two lactide - caprolactone copolymers that are different in the mole ratio from each other can be used as the above-mentioned copolymer. The copolymer to be used in the present invention may contain the above-mentioned copolymer alone or may contain another polymer or copolymer additionally in a range that does not affect the present invention.

The molecular weight (weight-average molecular weight) of the copolymer is not particularly limited but is, for example, 5,000 to 2,000,000, preferably 10,000 to 1,500,000, and more preferably 100,000 to 1,000,000. The mole ratio between lactide and caprolactone is, for instance, in the range of 90 : 10 to 10 : 90, preferably in the range of 85 : 15 to 20 : 80, and more preferably in the range of 80 : 20 to 40 : 60.

The method of preparing the copolymer is not particularly limited and conventionally well-known methods can be used. Generally, lactide and caprolactone that are used as starting materials may be copolymerized through ring-opening polymerization, or lactide (cyclic dimer of lactic acid) may be synthesized from lactic acid and then this may be copolymerized with caprolactone. The method of synthesizing lactide using lactic acid also is not particularly limited and conventionally well-known methods can be used. The aforementioned lactide is not particularly limited. L-lactide, D-lactide, or a mixture thereof (D, L-lactide) can be used as the lactide. On the other hand, L-lactic acid, D-lactic acid, or a mixture thereof (D,L-lactic acid) can be used as the lactic acid. When lactic acid is used as a starting material as described above, it is preferable that, with lactic acid that is a monomer being expressed in terms of lactide that is a dimer, the mole ratio between the lactide and caprolactone be in the above-mentioned ranges. Furthermore, examples of caprolactone include epsilon-caprolactone, gamma-caprolactone, delta-caprolactone, etc. Among them, epsilon-caprolactone is preferable.

In the present invention, the poor solvent and the good solvent each are not particularly limited, as long as the poor solvent is a solvent in which the polymer has a relatively low solubility and the good solvent is a solvent in which the polymer has a relatively high solubility and that is compatible with the solvent in which the polymer has a relatively low solubility. Generally, they can be determined according to the type of polymer used. Generally, examples of the poor solvent that can be used herein include water, ethanol, tertiary butyl alcohol (tBuOH), etc. On the other hand, generally, examples of the good solvent that can be used herein include organic solvents such as 1,4-dioxane, dimethyl carbonate, etc. that are compatible with the poor solvent. Particularly, a combination of water as the poor solvent and 1,4-dioxane as the good solvent is preferable.

Hereinafter, the process for producing a porous object of the present invention is described in detail. The method of adjusting the pore size is described later.

### <Process of Preparing Mixed Solution>

A polymer, a poor solvent, and a good solvent are mixed together and thereby a mixed solution is prepared. The order for adding the respective solvents is not particularly limited.

The concentration of the polymer in the mixed solution is not particularly limited but usually is in the range of 0.1 to 24 weight%, preferably in the range of 2 to 8 weight%, and more preferably in the range of 3 to 5 weight%. The ratio of the good solvent to be added to the mixed solution is determined suitably, for example, according to the amount of the poor solvent to be added, which is described later. Preferably, the weight ratio (polymer : good solvent) between the polymer and the good solvent is 0.1 : 99.9 to 24 : 76, more preferably 2 : 98 to 6 : 94, and particularly preferably 4 : 96.

The ratio of the poor solvent to be added to the mixed solution can be determined suitably according to the desirable pore size of a porous object to be formed and the constant cooling rate to be employed, as described later. The concentration of the poor solvent in the mixed solution is, for example, in the range of more than zero but not more than 20 weight%, preferably 0.1 to 20 weight%, more preferably 6 to 12.5 weight%, and particularly preferably 6 to 12.25 weight%. When the mixed solution has a polymer concentration of 3.6 weight%, the concentration of the poor solvent in the mixed solution is, for instance, in the range of more than zero but not more than 12.5 weight%, preferably 6 to 12.5 weight%. The weight ratio (polymer : poor solvent) between the polymer and the poor solvent is not particularly limited but is in the range of 3.2 : 20 to 4 : 0.5, for example.

### <Process of Freezing Treatment>

The mixed solution is cooled at a rate of 300°C/hr or lower and thereby is frozen. In the freezing process, no limitations are posed except that the mixed solution is cooled at a rate in the above-mentioned range. For instance, the mixed solution can be frozen using a commercial freeze-dryer. A preferable type of the freeze-dryer is one that can control the cooling rate. For example, TF5-85ATANCS (Trade Name; manufactured by Takara) can be used.

When the solution to be frozen is cooled, it is preferable that the mixed solution be placed in a container and then be cooled from the bottom of the container, for example. In this way, when it is cooled from the bottom of the container, the mixed solution can be cooled uniformly from the bottom toward the upper part at a constant rate and thus can be frozen uniformly and gradually Specifically, it is preferable that with a freezer or a freeze-dryer, the container including the mixed solution be placed on a cooling rack of the freezer and the temperature of the cooling rack be controlled to decrease at a constant rate of 300°C/hr or lower. In this manner, when the temperature of the cooling rack itself is allowed to decrease at the above-mentioned predetermined rate, the bottom of the container placed on the cooling rack can be cooled. This allows the mixed solution to be cooled from the bottom toward the upper part. The above-mentioned container is not particularly limited but can be a stainless steel container, for example.

The cooling rate is not particularly limited as long as it is 300°C/hr or lower. As described later, it can be determined suitably according to the desired pore size of the porous object to be formed and the concentration of the poor solvent in the mixed solution. The cooling rate is, for example, in the range of 3 to 300°C/hr, preferably in the range of 3 to 250°C/hr, more preferably in the range of 3 to 180°C/hr, and particularly preferably in the range of 5 to 180°C/hr. When a freezer is used as described above, the temperature of the cooling rack thereof can be controlled to decrease at a constant rate in such ranges (hereinafter the same applies).

The temperature of the mixed solution to be frozen is not particularly limited. For instance, it is equal to or higher than the freezing point of the solvent used, preferably in the range of 10°C to room temperature (for example, 20 to 37°C), and more preferably 10 to 20°C. Particularly, when cooling is started at a constant rate of 300°C/hr, it is preferable that the temperature of the mixed solution at the start of the freezing treatment be around 10°C. Furthermore, when the temperature of the mixed solution is set at around 10°C at the start of the freezing treatment as described above, it is preferable that in order to allow the whole mixed solution to be uniform (for instance, 10°C), the mixed solution be placed at a higher temperature (for example +10°C) than the temperature (for instance, 10°C) set at the above-mentioned start and then the temperature be reduced to that set at the start. In this case, the time required for reducing the temperature is not limited at all but can be approximately 60 minutes (or longer), for instance. When such a treatment is carried out before cooling is performed at a constant rate, the porous object can be produced with higher reproducibility.

The final temperature that is used for the freezing treatment is, for instance, the eutectic point or lower, preferably -10°C or lower, and more preferably in the range of -10 to -50°C. The final temperature that is used for the freezing treatment is not particularly limited. However, when it is set at around -10°C, the cost required for cooling can be reduced further.

When the temperature of the mixed solution has reached the final temperature that is used for freezing, the treatment at the final temperature that is used for freezing can be continued suitably according to the frozen state of the mixed solution. It may be continued until the mixed solution freezes completely. For example, it may be continued for longer than zero hour but not longer than 12 hours, preferably for about 1 to 3 hours.

The amount of the mixed solution to be freeze-dried is not particularly limited. The aforementioned conditions are particularly preferable conditions for the amount of the mixed solution whose depth is about 0.5 to 1 cm when it is placed in a container.

### <Process of Drying Treatment under Reduced Pressure>

The freeze-treated mixed solution obtained through the above-mentioned process of freezing treatment is dried under reduced pressure and thereby a porous object is obtained. The conditions for drying under reduced pressure are not limited. The drying under reduced pressure can be carried out by conventionally well-known methods.

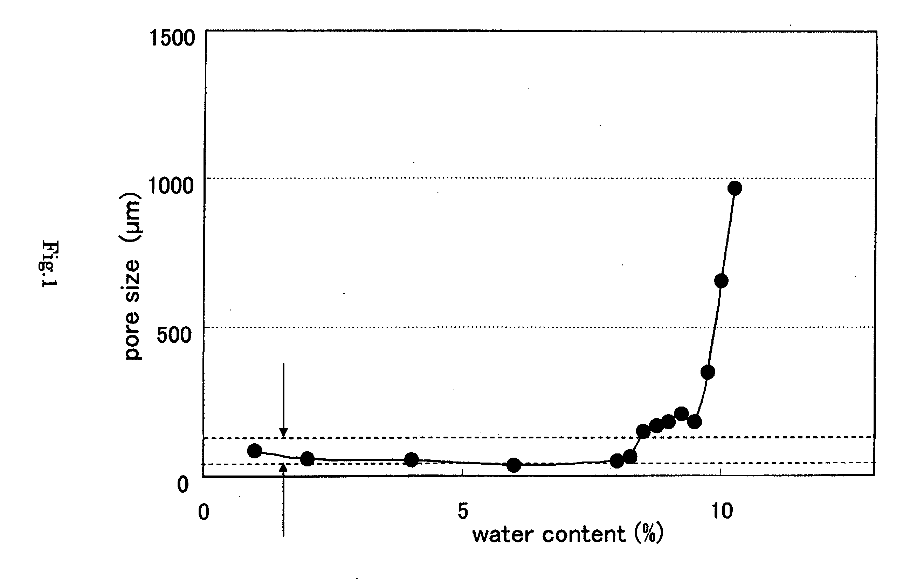

Next, the method of controlling the pore size of a porous object is described in detail. According to the control method of the present invention, for example, when a plurality of mixed solutions containing poor solvents whose concentrations are different from each other are freeze-treated at a constant cooling rate, the pore size varies according to the concentration of the poor solvent as shown in FIG. 1 described later. Furthermore, when various cooling rates are set, for example, the variations in pore size occur in the same manner at the respective cooling rates, i.e. the pore size increases in a certain range of the concentration of the poor solvent while decreasing in a certain range of the concentration. However, when the concentration remains the same, the pore size varies according to the cooling rate. That is, when not only the concentration of the poor solvent is varied but also the cooling rate is varied, pore sizes can be set over a wider range. Accordingly, for example, when a porous material is produced with the conditions of the cooling rate and the concentration of the poor solvent being varied and then a calibration curve is made that shows the relationship among the rate, concentration and pore size obtained, porous objects with desirable pore sizes in the range of about 30 to 1800 µm can be produced with high reproducibility.

In the case where the mixed solution contains the copolymer, good solvent, and poor solvent and the weight ratio between the copolymer and the good solvent is 4: 96, when, for example, the concentration of the poor solvent in the mixed solution and the cooling rate are set to satisfy the conditions indicated in the tables below, porous objects with pore sizes (30 to 1800 µm) indicated in the tables can be obtained.

**Table 1**

| | |
|---|---|
| Cooling Rate: 3°C/hr | |

| Concentration of Poor Solvent (weight%) | Pore Size (µm) |
|---|---|
| 6-9 | 30-200 |
| 9.25 - 9.75 | <200 - 400 |
| 10 | <400 - 800 |
| 10.25 | <800 - 1000 |
| Cooling Rate: 5°C/hr | |
| 6 - 9.5 | 30 - 200 |
| 4.75 - 10 | <200 - 400 |
| 10.25 - 10.5 | <400 - 800 |
| 10.75 | <800 - 1200 |
| 11 - 11.5 | <1200 - 1500 |
| Cooling Rate: 10°C/hr | |

| Concentration of Poor Solvent (weight%) | Pore Size (µm) |
|---|---|
| 6 - 10 | 30 - 200 |
| 10.25 | <200 - 400 |
| 10.5 - 10.75 | <400 - 800 |
| 11 | <800 - 200 |
| 11 - 11.75 | <1200 - 1800 |
| Cooling Rate: 180°C/hr | |

| Concentration of Poor Solvent (weight%) | Pore Size (µm) |
|---|---|
| 6 - 10.25 | 30 - 200 |
| 10.5 - 11 | <200 - 400 |
| 11.25 - 12 | <400 - 800 |

In the manner described above, the porous objects of the present invention can be obtained. The production process of the present invention allows pore sizes to be set in a wide range as described above. Accordingly, the porous objects of the present invention can be used for various uses depending on the pore sizes. Especially, when they are used as scaffold materials for cultured cells, those with relatively large pore sizes are preferable. For example, porous objects with pore sizes of 50 to 1000 µm, preferably pore sizes of 100 to 1000 µm, are useful. In addition, they can be used as various medical porous objects. The size and shape of the porous objects according to the present invention are not particularly limited. They can be determined according to the uses thereof.

The method of measuring the pore sizes of the porous objects according to the present invention is not particularly limited and a conventionally well-known method can be employed.

Hereinafter, the present invention is described further in detail using examples and comparative examples but is not limited thereto.

### <Example 1>

Porous objects were produced with mixed solutions whose water contents were different from each other, and thereby the control of pore sizes was checked.

A lactide-caprolactone copolymer (P(LA/CL = 50/50)), 1.4-dioxane, and water were mixed together, with the composition ratio (mole ratio) between L-lactide and epsilon-caprolactone being 50 : 50 in the lactide-caprolactone copolymer. Thus 29 mixed solutions were prepared that had different water contents. In this case, the mixing ratio (weight ratio) between P(LA/CL = 50/50) and 1,4-dioxane was constant (4 : 96), but the mixing ratio of water (water content) in each of the mixed solutions was changed to 1, 2, 4, 6, 8, 8.25, 8.5, 8,75, 9, 9.25, 9.5, 9.75, 10, 10.25, 10.5, 10.75, 11, 11.25, 11.5, 11.75, 12, 12.25, 12.5, 12.75, 13, 14, 16, 18, and 20 weight%. These mixed solutions each (20 g) were placed in a stainless steel petri dish (with a diameter of 5 cm and a depth of 1.5 cm; hereinafter the same applies).

The stainless steel petri dishes were placed on a cooling rack in a freeze-dryer (Trade Name: TF5-85ATANCS; manufactured by Takara) (room temperature). Then the cooling rack was set at 10°C and they were allowed to stand for one hour. Thereafter, the temperature of the cooling rack was cooled to -50°C at a rate of 3°C/hr, and then they were left to stand at -50°C for 180 minutes. The total period of time from the start of the treatment at 10°C to the end of the treatment at -50°C was 20 hours. Upon completion of the cooling treatment, the temperature inside the freeze-dryer was adjusted to 25°C and then the drying treatment was carried out under reduced pressure. Thus 29 porous object samples were produced.

Disk-shaped porous object samples each were taken out of the stainless steel petri dish and were cut in the middle in the thickness direction thereof. With respect to the cut surfaces, the pore sizes were measured by the following method (n = 5). The cut surface (0.5 cm²) of each porous object sample thus cut was observed with an electron microscope. A pore whose size was relatively large and had a higher appearance rate was selected from the whole cut surface, and the image obtained therefrom was analyzed with an image analysis software (NIH image). Thus, the pore sizes were computed.

### <Comparative Example 1>

A method of varying pore sizes depending on the setting of the temperature that is used for freezing was employed as a conventional method, and porous objects were produced by this method. The same P(LA/CL = 50/50) and 1.4-dioxane as those used in Example 1 were mixed together so that the weight ratio therebetween was 4 : 96. Thus, a mixed solution was prepared. This mixed solution (20 g) was placed in stainless steel petri dishes. Then the stainless steel petri dishes each were allowed to stand still in a freezer at a predetermined cooling temperature (-80, -30, or -15°C) for four hours and thereby the mixed solutions were frozen. Thereafter, these stainless steel petri dishes were placed in a freeze-dryer (Trade Name: Freeze dryer FDU-830; manufactured by EYELA, TOKYO RIKAKIKAI CO., LTD.) and drying was carried out under reduced pressure. In addition, the mixed solution (20 g) was placed in a stainless steel petri dish and then was frozen with liquid nitrogen (-196°C). Thereafter, drying was carried out under reduced pressure in the same manner. Thus, four porous object samples were produced. The pore sizes of the porous object samples thus obtained were indicated in Table 2 below.

### <Comparative Example 2>

A method of varying pore sizes depending on the polymer content was employed as a conventional method, and porous objects were produced by this method. The same P(LA/CL = 50/50) and 1.4-dioxane as those used in Example 1 were mixed together so that the weight ratios therebetween of respective portions were 2 : 98, 4 : 96, and 6 : 94. Thus mixed solutions were prepared. Then the drying was carried out under reduced pressure using the freeze-dryer (Trade Name: Freeze dryer FDU-830; manufactured by EYELA, TOKYO RIKAKIKAI CO., LTD.) in the same manner as in Comparative Example 1 described above except that the mixed solutions were frozen at -60°C using dry ice and ethanol. Thus porous object samples were produced. The pore sizes of the samples thus obtained are indicated in Table 2 below.

**Table 2**

| Comparative Example 1 | Temperature Used for Freezing | Pore Size (µm) |
|---|---|---|
| | -196°C | 12 |
| | -80°C | 33 |
| | -30°C | 56 |
| | -15°C | 82 |
| | | |

| Comparative Example 2 | Weight Ratio | Pore Size (µm) |
|---|---|---|
| | 2 : 98 | 83 |
| | 4 : 96 | 56 |
| | 6 : 94 | 46 |

FIG. 1 shows the relationship between the water content in the mixed solutions and the pore size of the samples. In FIG. 1, the range of pore sizes of the porous objects obtained in Comparative Examples 1 and 2 described above is indicated with dotted lines (the range indicated with arrows in FIG. 1). As shown in FIG. 1, it was found that the method of Example 1 made it possible to control pore sizes of porous objects and to form uniform pores, by varying the water content and cooling at a constant rate. Particularly, as shown in Table 2 above, Comparative Example 1 that employed a conventional method of adjusting pore sizes by changing the temperature used for freezing allows the pore sizes to be only approximately 10 to 80 µm, while Comparative Example 2 that employed a conventional method of adjusting pore sizes by changing the polymer content allows the pore sizes to be only approximately 40 to 80 µm. Comparative Examples 1 and 2 did not allow larger pores with a size of at least 90 µm to be formed. On the other hand, according to Example 1, pore sizes were obtained over a wide range of 30 to 1800 µm and were excellent in uniformity.

### Example 2

Porous objects were produced with cooling rates being varied. The control of pore sizes was checked.

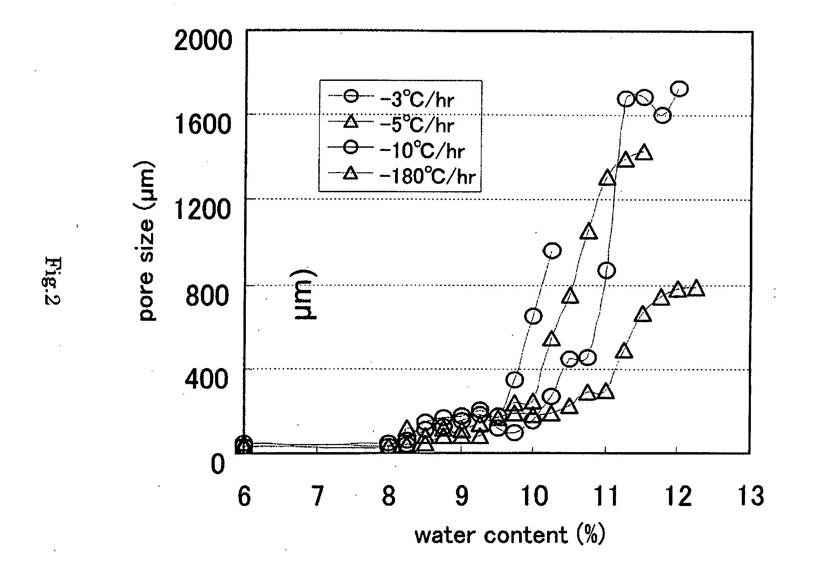

A plurality of mixed solutions whose water contents were different from each other were prepared in the same manner as in Example 1 except for using P (LA/CL = 51/49) in which the composition ratio between L-lactide and epsilon-caprolactone was 51 : 49. Then, the mixed solutions each (20 g) were placed in a stainless steel petri dish. The stainless steel petri dishes were placed on a cooling rack in the freeze-dryer (Trade Name: TF5-85ATANCS; manufactured by Takara). Then the temperature of the cooling rack was decreased to 10°C (over 25 minutes) and they were allowed to stand for 60 minutes. Thereafter, the cooling rack was cooled to -50°C at each of the predetermined rates (180°C/hr, 10°C/hr, 5°C/hr, and 3°C/hr) and then they were treated at -50°C for 180 minutes. Upon completion of the cooling treatment, the temperature inside the freeze-dryer was adjusted to 25°C and then drying treatment was carried out under reduced pressure. Thus porous object samples were produced. With respect to these porous object samples, the pore sizes were measured in the same manner as in Example 1 (n = 2). FIG. 2 shows the relationship between the water content in the mixed solutions and the pore size of the samples. It has been confirmed that the same behavior was exhibited when the same experiment as in Example 1 was carried out using the P(LA/CL) in which the composition ratio was 51:49.

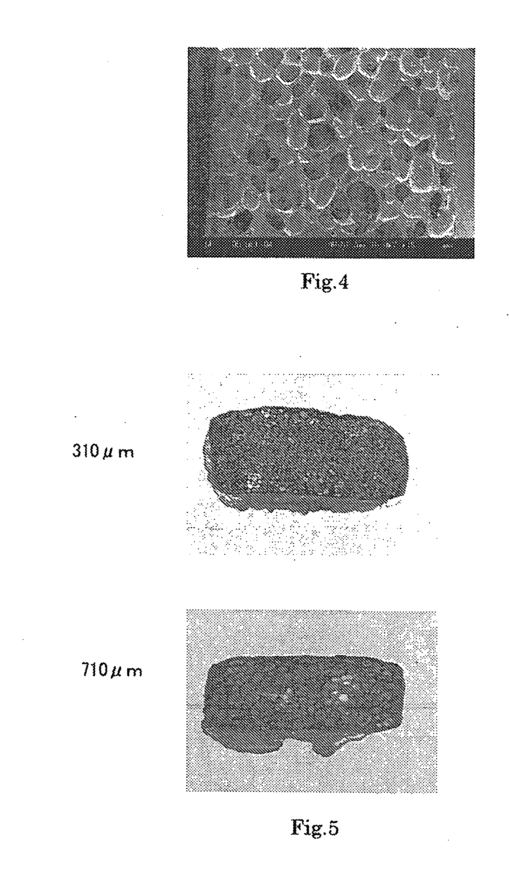

As shown in FIG. 2, regardless of which cooling rate was employed for cooling, the pore sizes of the porous objects varied with changes in water content, and the range of water contents that allowed the pore sizes to be particularly large also remained unchanged. In addition, even if mixed solutions have the same water content, the pore sizes can be varied by changing the cooling rate. A tendency could be seen in which the higher the cooling rate, the smaller the pore sizes, while the lower the cooling rate, the larger the pore sizes. Thus, it was found that adjustment of the water content and cooling rate made it possible easily to produce porous objects with desired pore sizes, particularly those with larger pore sizes that were difficult to produce by the conventional methods. FIG. 4 shows a photograph of the cross section of the porous object sample obtained at a cooling rate of 180°C/hr. As shown in FIG. 4, it can be seen that pores are distributed uniformly at the cross section and the sizes thereof also have excellent uniformity.

### Example 3

The influence of the variations in temperature used for freezing on pore sizes was checked.

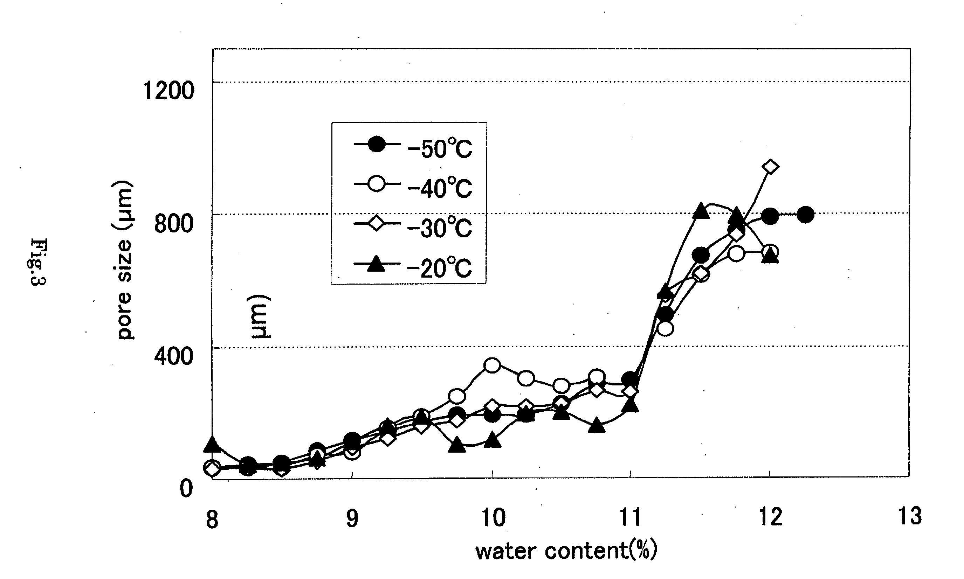

Porous object samples were produced in the same manner as in Example 2 except that the cooling rate was 180°C/hr and the final temperature used for cooling was set at predetermined temperatures (-20°C, -30°C, and -40°C). The pore sizes of the porous object samples were measured (n = 3). FIG. 3 shows the relationship between the water content in the mixed solutions and the pore size of the samples.

As shown in FIG. 3, when the cooling rate was 180°C/hr, the variations in the final temperature used for cooling did not cause considerable changes in pore sizes obtained corresponding to the water contents. Accordingly, it was found that the cooling temperature did not affect the pore sizes. Thus, it can be said that when the cooling temperature is set at -20°C, excessive cooling is no longer necessary and thereby the cost can be reduced.

### Example 4

Porous objects produced each were implanted in a rat body, and then the penetration of cells and tissues into the porous objects was checked.

Porous objects were produced in the same manner as in Example 1 using mixed solutions with predetermined water contents (8.5 weight%, 9.75 weight%, and 10.25 weight%). Then each porous object was cut into a size of 12 × 15 mm. Thus samples were prepared. The pore sizes of the samples obtained using the mixed solutions with water contents of 8.5 weight%, 9.75 weight%, 10.25 weight% were 130 µm, 310 µm, and 790 µm, respectively. The thickness of each sample was approximately 5 mm. These samples each were implanted subcutaneously in the dorsal region of a rat. Then the samples were subjected to hematoxylin-eosin staining (H-E staining) two weeks and four weeks thereafter. Thus, the state of penetration of the tissues into each sample was checked.

As a result, as shown in the photographs (four weeks) in FIG. 5, particularly considerable penetration of cells was observed in the porous object samples with larger pore sizes (310 µm and 790 µm). As described above, the production process of the present invention makes it possible to obtain porous objects with larger pore sizes that are suitable for carriers (scaffolds) of cells. Thus, it can be said that the production process of the present invention is very useful in medical fields.

### Industrial Applicability

As described above, the present invention allows pore sizes of porous objects to be set over a wider range. This allows pore diameters to be set according to the intended uses, such as scaffold materials for cells. Therefore the production process of the present invention can be said to be very useful in medical fields including regenerative medicine.

## Claims

1. A process for producing a porous object, the process comprising:
preparing a mixed solution containing:
a polymer containing a copolymer of lactide and caprolactone;
a solvent in which the polymer has a relatively low solubility being at least one selected from the group consisting of water, ethanol, tertiary butyl alcohol (tBuOH); and
a solvent in which the polymer has a relatively high solubility being at least one of 1,4-dioxane and dimethyl carbonate, freeze-treating the mixed solution,
wherein the mixed solution is cooled at a constant rate of 300°C/hr or lower
and thereby is frozen; and
drying the mixed solution that has been freeze-treated, under reduced pressure,
wherein a pore size of the porous object to be produced is controlled by varying the content of the solvent in which the polymer has a relatively low solubility in the mixed solution in the process of preparing the mixed solution, and
cooling the mixed solution at a constant rate of 300°C/hr or lower in the process of freeze-treating.

2. The process for producing a porous object according to claim 1, wherein in the process of freeze-treating, the mixed solution is placed in a container, and then the mixed solution is cooled from a bottom of the container.

3. The process for producing a porous object according to claim 2, wherein in the process of freeze-treating, a freezer is used for cooling the mixed solution, the container including the mixed solution is placed on a cooling rack of the freezer, and a temperature of the cooling rack is controlled to decrease at a constant rate of 300°C/hr or lower.

4. The process for producing a porous object according to claim 2, wherein the container is a stainless steel container.

5. The process for producing a porous object according to claim 1, wherein the process of freeze-treating employs a cooling rate in a range of 3 to 180°C/hr.

6. The process for producing a porous object according to claim 1, wherein the content of the solvent in which the polymer has a relatively low solubility in the mixed solution is in a range of 6 to 12.5 weight%.

7. The process for producing a porous object according to claim 1, wherein in the copolymer of lactide and caprolactone, the lactide and the caprolactone have a mole ratio in a range of 90 : 10 to 10 : 90.

8. The process for producing a porous object according to claim 1, wherein in the process of freeze-treating, a final temperature that is used for freeze-treating the mixed solution is an eutectic point or lower.

9. The process for producing a porous object according to claim 1, wherein in the process of freeze-treating, a final temperature that is used for freeze-treating the mixed solution is -10°C or lower.

10. The process for producing a porous object according to claim 9, wherein the final temperature that is used for freeze-treating the mixed solution is in a range of-50 to -10°C.

11. The process for producing a porous object according to claim 8, wherein in the process of freeze-treating, the mixed solution is treated at the final temperature that is used for freeze-treating the mixed solution for a range of longer than zero hour but not longer than 12 hours.

12. The process for producing a porous object according to claim 1, wherein the polymer has a concentration in a range of 0.1 to 24 weight% in the mixed solution.

13. The process for producing a porous object according to claim 1, wherein a weight ratio between the polymer and the solvent in which the polymer has a relatively high solubility is in a range of 0.1 : 99.9 to 24 : 76, optionally wherein the weight ratio between the polymer and the solvent in which the polymer has a relatively high solubility is 4 : 96.

14. The process for producing a porous object according to claim 1, wherein a weight ratio between the polymer and the solvent in which the polymer has a relatively low solubility is in a range of 3.2 : 20 to 4 : 0.5.

## Patentansprüche

1. Verfahren zur Herstellung eines porösen Gegenstandes, wobei das Verfahren Folgendes umfasst:
- Zubereiten einer vermischten Lösung, die Folgendes enthält:
• ein Polymer, das ein Copolymer von Laktid und Caprolacton enthält;
• ein Lösemittel, in dem das Polymer relativ gering-löslich ist, bei dem es sich um mindestens eines handelt, das aus folgender Gruppe ausgewählt ist: Wasser, Ethanol, tertiärer Butylalkohol (tBuOH); und
• ein Lösemittel, in dem das Polymer relativ hoch-löslich ist, bei dem es sich um mindestens eines von 1,4-Dioxan und Dimethylcarbonat handelt,
- Gefrierbehandeln der vermischten Lösung, wobei die vermischte Lösung mit einer konstanten Rate von 300 °C/h oder weniger gekühlt wird und dadurch gefroren wird; und
- Trocknen der vermischten Lösung, die gefrierbehandelt wurde, unter vermindertem Druck, wobei eine Porengröße des herzustellenden porösen Gegenstandes durch Variieren des Gehalts des Lösemittels, in dem das Polymer relativ gering-löslich ist, in der vermischten Lösung in dem Prozess der Zubereitung der vermischten Lösung gesteuert wird, und
- Kühlen der vermischten Lösung mit einer konstanten Rate von 300 °C/h oder weniger in dem Prozess der Gefrierbehandlung.

2. Verfahren zur Herstellung eines porösen Gegenstandes nach Anspruch 1, wobei die vermischte Lösung in dem Prozess der Gefrierbehandlung in einen Behälter gegeben wird und dann die vermischte Lösung vom Boden des Behälters her gekühlt wird.

3. Verfahren zur Herstellung eines porösen Gegenstandes nach Anspruch 2, wobei in dem Prozess der Gefrierbehandlung ein Gefrierschrank zum Kühlen der vermischten Lösung verwendet wird, wobei der Behälter, der die vermischte Lösung enthält, auf eine Kühlablage des Gefrierschranks gestellt wird und eine Temperatur der Kühlablage so gesteuert wird, dass sie mit einer konstanten Rate von 300 °C/h oder weniger abnimmt.

4. Verfahren zur Herstellung eines porösen Gegenstandes nach Anspruch 2, wobei der Behälter ein Edelstahlbehälter ist.

5. Verfahren zur Herstellung eines porösen Gegenstandes nach Anspruch 1, wobei der Prozess der Gefrierbehandlung mit einer Kühlrate in einem Bereich von 3 bis 180 °C/h ausgeführt wird.

6. Verfahren zur Herstellung eines porösen Gegenstandes nach Anspruch 1, wobei der Gehalt des Lösemittels, in dem das Polymer relativ gering-löslich ist, in der vermischten Lösung in einem Bereich von 6 bis 12,5 Gewichts-% liegt.

7. Verfahren zur Herstellung eines porösen Gegenstandes nach Anspruch 1, wobei in dem Copolymer von Laktid und Caprolacton das Laktid und das Caprolacton ein MolVerhältnis in einem Bereich von 90:10 bis 10:90 haben.

8. Verfahren zur Herstellung eines porösen Gegenstandes nach Anspruch 1, wobei in dem Prozess der Gefrierbehandlung eine endgültige Temperatur, die zur Gefrierbehandlung der vermischten Lösung verwendet wird, ein eutektischer Punkt oder niedriger ist.

9. Verfahren zur Herstellung eines porösen Gegenstandes nach Anspruch 1, wobei in dem Prozess der Gefrierbehandlung eine endgültige Temperatur, die zur Gefrierbehandlung der vermischten Lösung verwendet wird, -10 °C oder niedriger ist.

10. Verfahren zur Herstellung eines porösen Gegenstandes nach Anspruch 9, wobei die endgültige Temperatur, die zur Gefrierbehandlung der vermischten Lösung verwendet wird, in einem Bereich von -50 bis -10 °C liegt.

11. Verfahren zur Herstellung eines porösen Gegenstandes nach Anspruch 8, wobei die vermischte Lösung in dem Prozess der Gefrierbehandlung bei der endgültigen Temperatur, die zur Gefrierbehandlung der vermischten Lösung verwendet wird, über eine Zeitspanne von mehr als null Stunden, aber nicht länger als 12 Stunden behandelt wird.

12. Verfahren zur Herstellung eines porösen Gegenstandes nach Anspruch 1, wobei das Polymer eine Konzentration in einem Bereich von 0,1 bis 24 Gewichts-% in der vermischten Lösung hat.

13. Verfahren zur Herstellung eines porösen Gegenstandes nach Anspruch 1, wobei ein Gewichtsverhältnis zwischen dem Polymer und dem Lösemittel, in dem das Polymer relativ hoch-löslich ist, in einem Bereich von 0,1:99,9 bis 24:76 liegt, wobei optional das Gewichtsverhältnis zwischen dem Polymer und dem Lösemittel, in dem das Polymer relativ hoch-löslich ist, 4:96 beträgt.

14. Verfahren zur Herstellung eines porösen Gegenstandes nach Anspruch 1, wobei ein Gewichtsverhältnis zwischen dem Polymer und dem Lösemittel, in dem das Polymer relativ gering-löslich ist, in einem Bereich von 3,2:20 bis 4:0,5 liegt.

## Revendications

1. Procédé pour produire un objet poreux, ledit procédé comprenant :
- la préparation d'un mélange de solution qui continent :
• un polymère comportant un copolymère de lactide et de caprolactone ;
• un solvant dans lequel le polymère présente une solubilité assez faible, le solvant étant au moins l'un des éléments choisis parmi le groupe suivant : eau, éthanol, alcool butylique tertiaire (tBuOH) ; et
• un solvant dans lequel le polymère présente une solubilité assez élevée, le solvant étant au moins l'un des éléments suivants : 1,4-dioxane et carbonate de diméthyle,
- le traitement à froid du mélange de solution, dans lequel le mélange de solution étant refroidi à un taux constant de 300 °C/h ou moins, et est donc congelé ; et
- le séchage à pression réduite le mélange de solution qui a été traité à froid, dans lequel la taille des pores de l'objet poreux qu'il s'agit de produire est contrôlée en faisant varier la teneur du solvant dans lequel le polymère a une solubilité assez faible dans le mélange de solution au cours de l'opération consistant à préparer le mélange de solution et
- le refroidissement du mélange de solution à un taux constant de 300 °C/h ou moins au cours de l'opération de traitement à froid.

2. Procédé pour produire un objet poreux selon la revendication 1, dans lequel, au cours de l'opération de traitement à froid, le mélange de solution est placé dans un récipient puis le mélange de solution est refroidi par le fond du récipient.

3. Procédé pour produire un objet poreux selon la revendication 2, dans lequel, au cours de l'opération de traitement à froid, un congélateur est utilisé pour refroidir le mélange de solution, le récipient incluant le mélange de solution étant placé sur une grille de refroidissement du congélateur et la température de la grille de refroidissement est contrôlée pour baisser à un taux constant de 300 °C/h ou moins.

4. Procédé pour produire un objet poreux selon la revendication 2, dans lequel le récipient est un récipient en acier inoxydable.

5. Procédé pour produire un objet poreux selon la revendication 1, dans lequel l'opération de traitement à froid utilise un taux de refroidissement compris entre 3 et 180 °C/h.

6. Procédé pour produire un objet poreux selon la revendication 1, dans lequel la teneur du solvant dans lequel le polymère présente une solubilité relativement faible dans le mélange de solution est compris entre 6 et 12,5 % en poids.

7. Procédé pour produire un objet poreux selon la revendication 1, dans lequel le copolymère de lactide et de caprolactone ont un taux molaire compris entre 90 _{:} 10 et 10 : 90 dans le copolymère de lactide et de caprolactone.

8. Procédé pour produire un objet poreux selon la revendication 1, dans lequel, au cours de l'opération de traitement à froid, la température finale utilisée pour le traitement à froid du mélange de solution est un point eutectique ou inférieure à celui-ci.

9. Procédé pour produire un objet poreux selon la revendication 1, dans lequel, au cours de l'opération de traitement à froid, la température finale utilisée pour le traitement à froid du mélange de solution est de - 10 °C ou moins.

10. Procédé pour produire un objet poreux selon la revendication 9, dans lequel la température finale utilisée pour le traitement à froid du mélange de solution est comprise entre -50 et -10 °C.

11. Procédé pour produire un objet poreux selon la revendication 8, dans lequel, au cours de l'opération de traitement à froid, le mélange de solution est traité à la température finale utilisée pour le traitement à froid du mélange de solution pendant une durée supérieure à zéro heure mais ne dépassant pas 12 heures.

12. Procédé pour produire un objet poreux selon la revendication 1, dans lequel le polymère présente une concentration comprise entre 0,1 et 24 % en poids dans le mélange de solution.

13. Procédé pour produire un objet poreux selon la revendication 1, dans lequel le rapport en poids entre le polymère et le solvant dans lequel le polymère a une solubilité relativement élevée est compris entre 0,1 : 99,9 et 24 : 76, optionnellement le rapport en poids entre le polymère et le solvant dans lequel le polymère a une solubilité relativement élevée est 4 : 96.

14. Procédé pour produire un objet poreux selon la revendication 1, dans lequel le rapport en poids entre le polymère et le solvant dans lequel le polymère a une solubilité relativement faible est compris entre 3,2 : 20 et 4 : 0,5.
